# EUROPEAN PATENT SPECIFICATION

(11) **EP 2 208 483 B1**
(45) Date of publication and mention of the grant of the patent: **02.12.2015**
(21) Application number: 10150688.9
(22) Date of filing: 13.01.2010
(51) Int. Cl.: A61F 2/915, A61F 2/95, A61F 2/966

(54) **Delivery apparatus for a retractable self expanding neurovascular stent and its use**
Abgabevorrichtung für einen zurückziehbaren, selbstaufweitenden neurovaskulären Stent und dessen Verwendung
Appareil de mise en place pour endoprothèse neurovasculaire rétractable auto-étendue et son utilisation

(30) Priority: 19.01.2009 CN 200910045521
(43) Date of publication of application: 21.07.2010
(73) Proprietor: Achieva Medical (Shanghai) Co., Ltd., Pudong, Shanghai 201203 (CN)
(72) Inventor: Zhang, Yi, San Diego, CA 92128 (US)
(74) Representative: Tombling, Adrian George

(56) References cited:
- EP-A1- 1 518 515
- EP-A2- 1 982 677
- WO-A1-93/11825
- WO-A1-2004/008991
- WO-A1-2009/149457
- WO-A2-2007/088549
- WO-A2-2008/028964
- US-A- 6 120 522
- US-A1- 2003 163 193
- US-A1- 2004 059 407

## Description

The present invention relates to a neurovascular stent delivery apparatus for delivering a self-expanding neurovascular stent to a neurovascular target site, that allows for smooth movement of the apparatus along a typically tortuous vascular path, ease of stent deployment, and ease of stent retractability being pushed and pulled through the delivery apparatus.

It is well documented that annually in the United States, approximately 780,000 patients will experience new or recurring stroke. Stroke affects the arteries leading to and within the brain. A stroke occurs when a blood vessel that carries oxygen and nutrients to the brain is either blocked by intracranial atherosclerotic clot (ischemic stroke) or bursts (hemorrhagic stroke). When that happens, part of the brain cannot get the blood (and oxygen) it needs, so it starts to die. Of these strokes, approximately 88% are diagnosed as ischemic in nature, while the remaining 12% are attributable to hemorrhagic events.

Successful stenting for the management of coronary atherosclerotic stenosis has sparked intense interest in research and development of neurovascular stents for intracranial indications. Safety of balloon-expandable stenting is often compromised by limited flexibility of the balloon-mounted stent delivery systems, high inflation pressure required to deploy stent in fragile intracranial vessels, the risk of shearing the stent off the balloon while navigating to the target lesion, and by difficulty in accurately sizing the stent to the vessel diameter. Balloon-expandable stents have been largely replaced by self-expanding stent technology, because of self-expanding stent's ease of use, better deliverability, and lower tendency for vessel rupture and damage to the artery during deployment. (*See e*.*g*., Higashida RT, Tsai FY, Halbach VV, et al. Transluminal angioplasty, thrombolysis, and stenting for extracranial and intracranial cerebral vascular disease. J Intervent Cardiol 1996;9:245-256; Han PP, Albuquerque FC, Ponce FA, et al. Percutaneous intracranial stent placement for aneurysms. J Neurosurg 2003;99:2330; Lylyk P, Ceratto R, Hurvitz D, Basso A. Treatment of a vertebral dissecting aneurysm with stents and coils. Neurosurgery 1998;43:385-388; Lylyk P, Cohen JE, Ceratto R, et al. Endovascular reconstruction of intracranial arteries by stent placement and combined techniques. J Neurosurg 2002;97:1306-1313; and Mericle RA, Lanzino G, Wakhloo AK, et al. Stenting and secondary coiling of intracranial internal carotid artery aneurysm. Neurosurgery 1998;43:1229-1234). Although a variety of intravascular self-expanding stents have been proposed heretofore, for example, in U.S. Pat. No. 4,665,906 issued to Jervis and U.S. Pat. No. 4,925,445 to Sakamoto et al, designing delivery systems for delivering self-expanding stent has proven difficult. Accordingly, the prior art teaches various methods and apparatuses for delivering the self-expanding stents. Examples of such delivery methods and apparatuses can be found in U.S. Pat. No. 4,580,568 issued to Gianturco; U.S. Pat. No. 7,169,170 issued to Widenhouse; and U.S. Pat. No. 7,311,726 issued to Mitelberg et al.

U.S. Pat. No. 4,580,568 discloses a delivery apparatus which uses a hollow sheath, like a catheter. The sheath is inserted into a body vessel and navigated there-through so that its distal end is adjacent the target site. A self-expanding stent is then compressed to a smaller diameter and loaded into the sheath at the sheath's proximal end. A cylindrical flat end pusher, having a diameter almost equal to the inside diameter of the sheath is inserted into the sheath behind the stent. The pusher is then used to push the stent from the proximal end of the sheath to the distal end of the sheath. Once the stent is at the distal end of the sheath, the sheath is pulled back, while the pusher remains stationary, thereby exposing the stent and allowing it to expand within the vessel. However, delivering the stent through the entire length of the catheter may cause many problems, including possible damage to a vessel or the stent during its travel. The small and tortuous neurovascular pathway makes this system even more problematic because the pathway increases the risk of damage to the vessels and/or stent. U.S. Pat. No. 7,169,170 describes a prior art pre-loaded self expanding stent delivery system wherein the stent is pre-loaded into the distal end of the outer catheter and the outer catheter and preloaded stent are simultaneously delivered through a pathway and to a target site. This patent discloses a delivery apparatus to deliver the pre-loaded self-expanding stent to a target site. Once the physician determines that the stent is sufficiently placed about the target site, the outer sheath is pulled back proximately, while the inner shaft is held in a fixed position. The stop on the inner shaft prevents the stent from sliding back with the outer sheath, so as the sheath is moved back, the stent is effectively "pushed" out of the distal end of the sheath. But, frequently these preloaded stents are misdeployed, meaning that the stent is not at the optimal position at the target site, because of either less that optimal placement of the outer catheter to the target site and/or because of movement of the apparatus during the deployment.

One important factor for controlled delivery of the stent is having a precisely controlled retraction of the retractable outer sheath. Some of the technical difficulties of the known delivery apparatus are misdeployment of the self-expanding stent if it was either too distal or too proximal to the target lesion. Accordingly, the prior art teaches various methods and apparatuses for repositioning a self-expanding stent. Examples of such methods and apparatuses can be found in U.S. Pat. No. 6,267,783 issued to Leterdre et al.; U.S. Pat. No. 6,843,802 issued to Villalobos et al., U.S. Pat. No. 7,001,422 issued to Escamilla et al., and U.S. Pat. No. 7,311,726 issued to Mitelberg et al.

The delivery system in the above referenced Escamilla patent includes proximal, intermediate and distal cylindrical members disposed on and spaced apart along an elongated core wire such that first and second gaps are formed. The expandable stent includes anchor members which align with the gaps. The expandable stent is mounted on the intermediate cylindrical member, and the anchor members are disposed within the gaps thereby locking the stent onto the core member. In this configuration, the self-expanding stent can be pushed and pulled through the deployment catheter without damaging or deforming the stent. However, the Escamilla devices do not work well for neurovascular arteries because the elongated core is solid, the guide wire normally needed for interventional procedure can not be inserted through the device, so the elongated core wire must function as guide wire as well. It has proven difficult to design a core wire member having enough flexibility to navigate through tortuous neurovascular vessels as the guiding wire, but also enough stiffness to push the stent out of the catheter or pull the stent back into the catheter. Furthermore, the stent, in a constrained state, being mounted on the intermediate cylindrical member, may be damaged or deformed by the intermediate cylindrical member during advancement to the target disease site or during stent deployment.

Devices, such as the one shown in the above referenced Villalobos patent, use a method to reposition the stent as necessary to properly align with the target lesion to treat abdominal aortic aneurysms. The stent delivery apparatus comprises an inner shaft, outer sheath and self-expanding stent. The inner shaft has a distal end and a proximal end, the inner shaft distal end further includes at least two grooves; the self-expanding stent has a distal end and a proximal end, the self-expanding stent further includes at least two spaced apart longitudinal legs, the legs extend proximally away from the self-expanding stent, each leg including a flange. The flanges are set within the inner shaft grooves. The flange-groove combination allows the physician to partially deploy the stent while the flanges remain within the sheath, and allows physicians to pull the stent back into the delivery device if the placement is not optimal.

While the Villalobos device is designed for large vessels such as abdominal aortic, since the outer shaft size is in the range from 18F to 24 F (6mm-8mm). However, neurovascular arteries are generally very tortuous and quite small, having a diameter ranging from 2.0mm to 4.0mm in the Circle of Willis, 2.5 to 5.5mm in the cavernous segment of the internal carotid artery, 1.5 to 3.0mm in the vessels of the distal anterior circulation, and 2.0 to 4.0mm in the posterior circulation, the stent is placed at the target site via a small-diameter catheter having a lumen inner diameter of 0.7mm or less and out diameter of 3F (1mm) or less. For such small size scale, it is not easy to fabricate the devices, neither assemble the devices together. Moreover, cutting such grooves into the inner shaft of a small neurovascular delivery apparatus will result in a delivery apparatus that is too weak for delivery through a tortuous pathway and that will be easily torn under the required stresses for delivery and retractable deployment of the stent. Devices disclosed in U.S. Pat. No. 6,267,783 and U.S. Pat. No. 7,311,726 have the similar disadvantages.

Accordingly, there has been a desire to have a mechanism for preventing a stent being sheared off the inner shaft while navigating to a neurovascular target site. There has been a desire to have an improved mechanism to allow a neurovascular stent to be easily pushed out of and retrieved into the small diameter delivery apparatus if the placement is not optimal. There has been a desire to have a better device to prevent the stent from being damaged or deformed during advancement through a tortuous vascular path. The following described invention provides such an improved device.

The current invention overcomes the deficiencies of the prior art to provide a delivery apparatus enabling a self-expanding stent that is retractable during stent placement in the target neurovascular site. A conventional guide wire is preferably used in conjunction with the invention, thus the apparatus is configured to accept a guide wire. The apparatus includes an outer catheter, an inner shaft located coaxially within the outer catheter and a self-expanding stent, mounted onto the inner shaft and preloaded in its contracted state within the outer catheter's distal region. In one embodiment, the outer catheter has an outer diameter that is from about 0.94 mm (0.037 inch) to about 0.99 mm (0.039 inch). In one embodiment, the inner shaft has an outer diameter of about 0.56 mm (0.022 inch) to about 0.62 mm (0.0245 inch) for the middle and distal sections and an outer diameter of about 0.51 mm (0.02 inch) to about 0.52 mm (0.0205 inch) for the distal portion. In a preferred embodiment, the inner shaft has an inner diameter to allow passage of the guide wire, the inner diameter being of a sufficient size for allowing the passage of a 0.36 mm (0.014 inch) or smaller diameter guide wire. In a most preferred embodiment, the inner diameter of the inner shaft is about 0.43 mm (0.017 inch). The inner shaft includes at least one stent blocking member disposed in the distal section. The stent is mountable on the distal section of the inner shaft and preloaded within the outer catheter distal region. The self-expanding stent has proximal, middle and distal ends and is comprised of a plurality of closed cells. The closed cells may be in contact with the teeth of a gear-shaped blocking member disposed on the inner shaft so that the teeth of the gear protrude through the open space portion of a cell and can come in contact with the material portion, generally referred to as a strut, of a cell. The self-expanding stent may include locking members which interlock with a gap region formed by two or more blocking member(s) disposed on the inner shaft so as to lock the stent onto the inner shaft within the outer catheter. The stent and inner shaft are within the outer catheter and are arranged one to the other so as to enable the stent to be retractably moved out of and retrieved back to the outer catheter during placement at a target site. The invention may be used in the treatment of blood vessel blockage and aneurysms which occur in the brain. A combination of these configurations may be used, as well.

Preferably, the stent locking members are radiopaque and made of radiopaque materials, such as a metal, a metal alloy, a radiopaque polymer, gold, silver, platinum, an alloy containing gold, an alloy containing silver, an alloy containing platinum or an alloy containing one or more of gold, silver and platinum. Types of radiopaque materials are known in the art, as are their uses with stent delivery. The thickness of the stent locking member shall be slightly greater than stent strut thickness. Stent cells open area are preferably larger at the ends than in the middle, in order for stent to be better interlocked with a gear shaped blocking member on the inner shaft. To treat wide neck aneurysm, preferably, the stent is covered with graft material in the middle, but not on the ends. Graft in the middle of the stent will efficiently cover aneurysm wide neck, while the stent end without being graft covered will be interlocked with the blocking members on the inner shaft. Stent graft material is made of materials such as e-PTFE, Polyurethane, etc, as is known in the art.

The material composition and construction of the outer catheter changes over the length of it to create three distinct stiffness regions: proximal, middle, and distal. The inner shaft has also three distinct sections along the length of it. The variation of the stiffness from proximal end to distal end is to achieve better trackability inside the tortuous neurovascular path. As mentioned, the inner shaft includes at least one blocking member, to prevent any relative movement between the inner shaft and stent during advancement to the target neurovascular site, and also to allow retractable delivery of the stent to the target site, wherein physicians "pull" the stent back into the delivery apparatus if the placement is not optimal. Preferably, the inner shaft has reduced diameter inner member at the distal region, so as to be extending through the contracted stent interior, to enable stent and the inner shaft to be as an integral part during movement within the outer catheter.

In an embodiment of the inner shaft, the distal section further comprises at least one blocking member, and preferably the at least one blocking member is fixedly attached to the distal section of the inner shaft. The fixedly attached blocking member can be formed as a disc or as a gear with a plurality of teeth; the number of gear teeth preferably ranging from 2 to 8. The gear teeth interlock with stent cells by fitting within the open spaces formed by the struts. The number of gear teeth will be varied to accommodate different stent cell designs. For instance, gear with 2, 3 or 6 teeth could be used for six crown design stent, gear with 2 or 4 teeth could be used for eight crown design stent. Thus, the gear shaped blocking member will be configured to interlock with a compressed self-expanding stent that is to be loaded on the inner shaft for delivery at a target site. Alternatively, the at least one blocking member is formed as a disc at the distal end of the inner shaft, and an additional blocking member is formed by varying the outer diameter of the distal section of the inner shaft.

Thus, in an embodiment, there is provided a neurovascular stent delivery apparatus, wherein said delivery apparatus comprises the inner shaft of the current invention and further comprises an outer catheter and a pre-loaded self-expanding stent. Preferably, the self-expanding stent is preloaded into the distal section of the outer catheter and is configured on the distal section of said inner shaft such that the stent is interlocked with the blocking member(s) portion of the inner shaft. In one aspect, the inner shaft comprises at least one blocking member. In one aspect, the inner shaft comprises a disc-shaped blocking member. In one aspect, the inner shaft comprises a gear shaped blocking member. In one aspect, the inner shaft comprises at least two blocking members configured along said inner shaft to form a gap section. In one aspect, a gear-shaped blocking member is positioned such that the teeth of said gear-shaped blocking member stick through the open space of the stent cells. In one aspect, a locking member on the self-expanding stent is positioned between two blocking members on the inner shaft configured to form a gap that accommodates the locking member.

One embodiment of the invention provides a neurovascular stent delivery apparatus comprising an inner shaft having a proximal portion, a middle portion and a distal portion, wherein a first blocking member is fixedly attached on the distal section, and the second blocking member is formed by varying the outer diameter of the middle and distal sections of the inner shaft so that the middle section is larger that the distal section, said first and second blocking members being configured to form a gap that interlocks with a self expanding stent comprising at least one locking member when said self-expanding stent is compressed for loading into said neurovascular stent delivery apparatus.

In one aspect of the neurovascular stent delivery apparatus, the first blocking member is disc-shaped. In one aspect of the neurovascular stent delivery apparatus, the first blocking member is gear-shaped, comprising a plurality of teeth to interlock with the cells of said compressed self expanding stent. In an aspect, the plurality of teeth is at least 2 teeth. In an aspect, the plurality of teeth is selected from the group consisting of 2 teeth, 3 teeth, 4 teeth, 5 teeth, 6 teeth, 7 teeth and 8 teeth.

In one aspect of the neurovascular stent delivery device, the first blocking member is made of a radiopaque material. In an aspect, the radiopaque material is a metal, a metal alloy, or a radiopaque polymer. In an aspect, the radiopaque material is gold, silver, platinum or an alloy containing one or more of any of the aforementioned. In one aspect of the neurovascular stent delivery apparatus, the locking member is a radiopaque material.

In one aspect of the neurovascular stent delivery apparatus, the self-expanding stent is covered with a graft material.

In one aspect of the neurovascular stent delivery apparatus wherein the first blocking member is a gear shape, the cells of the self-expanding stent are larger at the proximal and distal ends of the self-expanding stent compared to the middle section cells.

In one aspect of the neurovascular stent delivery apparatus, the inner shaft is made of a material of varying hardnesses, wherein its distal end is softest.

Another embodiment of the invention provides a neurovascular stent delivery apparatus comprising an inner shaft having a proximal end, a middle end and a distal end, wherein said middle end is configured with a gear-shaped blocking member comprising 2 or more teeth to interlock with the cells of a compressed self-expanding stent, when said stent is loaded into said neurovascular stent delivery apparatus. In one aspect of the neurovascular stent delivery apparatus, the gear shaped blocking member has 2 teeth, 3 teeth, 4 teeth, 5 teeth, 6 teeth, 7 teeth or 8 teeth.

In one aspect of the neurovascular stent delivery apparatus the gear shaped blocking member is made of a radiopaque material. In an aspect, the radiopaque material is selected from the group consisting of: a metal, a metal alloy, a radiopaque polymer, gold, silver, platinum and an alloy containing one or more of gold, silver or platinum.

In one aspect of the neurovascular stent delivery apparatus, the self expanding stent comprises a radiopaque material. In one aspect of the neurovascular stent delivery apparatus, the self expanding stent is covered with a graft material. In one aspect of the neurovascular stent delivery apparatus, the cells of the self-expanding stent are larger at the proximal and distal ends of said self-expanding stent compared to the middle section cells.

The neurovascular stent delivery apparatus described above can be used in a method for retractably delivering a self-expanding stent to a neurovascular target site, the method comprising the steps of: a.) obtaining a neurovascular delivery apparatus containing a preloaded and compressed self expanding stent, wherein said preloaded and compressed self expanding stent is interlocked with an inner shaft member of said neurovascular delivery apparatus; b.) navigating a neurovascular path towards a neurovascular target site; c.) partially deploying the preloaded and compressed self-expanding stent by pulling the outer catheter to partially expose the stent out of the distal end of an outer catheter member of a neurovascular delivery device; d.) optionally, pulling the preloaded and compressed self expanding stent back inside of said outer catheter distal end; and e.) fully deploying the preloaded and compressed self-expanding stent to the target site by pulling the outer catheter to expose the stent out of the distal end of an outer catheter member of a neurovascular delivery device.

In one aspect of the method, the neurovascular path includes one of more of a neurovascular artery, the Circle of Willis, the cavernous section of the internal carotid artery, the vessels of the distal anterior circulation and the vessels of the posterior circulation.

In one aspect of the method, the neurovascular delivery apparatus comprises an inner shaft member having a first blocking member and a second blocking member configured along said inner shaft member to form a gap section that accepts the locking member of said preloaded and compressed self expanding stent. In an aspect, the stent is partially deployed from the outer catheter distal end by applying a force to the outer catheter that causes the second blocking member to contact the locking member of the stent. In an aspect, the partially deployed stent is retracted back inside of the outer catheter by applying a force to the inner shaft that causes the first blocking member to contact the locking member of the stent and translocate the stent in a direction that is coaxal to the outer catheter lumen.

In one aspect of the method, the stent is fully deployed from the outer catheter distal end by applying a force to the outer catheter that causes the second blocking member to contact the locking member of the stent until the stent is completely outside of the outer catheter, wherein the stent expands thereby removing the locking member from the proximity of the blocking members.

In one aspect of the method, one or more of said first and second blocking members and said locking member is made from a radiopaque material.

In one aspect of the method, the neurovascular delivery apparatus comprises an inner shaft member having a first blocking member configured as a gear with a plurality of teeth that interlocks with the cells of said preloaded and compressed self expanding stent. In an aspect, the plurality of teeth is at least 2. In an aspect, the plurality of teeth is selected from the group consisting of 2, 3, 4, 5, 6, 7 and 8.

In one aspect of the method, the stent is partially deployed from the outer catheter distal end by applying a force to the outer catheter that causes the plurality of teeth to contact the strut portion of the cell. In one aspect of the method, the partially deployed stent is retracted back inside of the outer catheter by applying a force to the inner shaft that causes the plurality of teeth to contact the strut portion of the cell and translocate the stent in a direction that is coaxal to the outer catheter lumen. In one aspect of the method, the stent is fully deployed from the outer catheter distal end by applying a force to the outer catheter that causes the plurality of teeth to contact the strut portion of the cell until the stent is completely outside of the outer catheter, wherein the stent expands thereby removing the cells from the proximity of the gear teeth.

In one aspect of the method, the cells of the self-expanding stent are larger at the proximal and distal ends of the self-expanding stent compared to the middle section cells. In one aspect of the method, one or more of the first blocking member and a portion of the stent is made from a radiopaque material. In one aspect of the method, the stent is covered with a graft material.

In one aspect of the method, either or both of the outer catheter and the inner shaft comprise a plurality of sections of differing hardnesses; the distal end being the softest. In one aspect of the method, the inner shaft has a smaller outer diameter at its distal end than the outer diameter of the remainder of the inner shaft.

One embodiment of the invention provides the neurovascular stent delivery apparatus described above for use in therapy.

Another embodiment of the invention provides the neurovascular stent delivery apparatus described above for use for retractably delivering a self-expanding stent to a neurovascular target site.

As will be appreciated by one skilled in the art, the method described above is equally applicable to the use of the neurovascular stent delivery apparatus. Further, certain aspects of the method will also be applicable to the delivery apparatus.

The foregoing and other aspects of the present invention will best be appreciated with reference to the detailed description of the invention in conjunction with the accompanying drawings, wherein:
FIG. 1 is a side view of a preferred embodiment of the delivery apparatus and a stent for retractable deployment at a target neurovascular site (not shown in FIG. 1).
FIG. 2 is a side view of another preferred embodiment of the delivery apparatus and a stent for retractable deployment at a target neurovascular site (not shown in FIG. 2).
FIG. 3 is a simplified elevational view of one preferred embodiment of the first and second blocking members disposed on the distal inner shaft, wherein the first blocking member is a disc shape.
FIG. 4 is a simplified elevational view of another preferred embodiment of the first blocking member disposed on the inner shaft, wherein the first blocking member is a gear shape.
FIG. 5 is a simplified elevational view of another preferred embodiment of the first blocking member disposed on the inner shaft, wherein the first blocking member is a gear shape.
FIG. 6 is a simplified elevational view of another preferred embodiment of the first blocking member disposed on the inner shaft, wherein the first blocking member is a gear-shape.
FIG. 7 is partial cross-sectional views showing the deployment of the closed-cell self-expanding stent with stent marker placement.
FIG. 8 is partial cross-sectional views of another preferred embodiment showing the deployment of the closed-cell self-expanding stent with stent marker placement.
FIG. 9 is side view of inner shaft.
FIG. 10 is side view of outer catheter.

As is used herein, the terms "about" or "approximate" when used to describe the dimensions of the described device mean that the size of the device need not be precisely the dimensions described. Those of skill in the art will understand from this disclosure how to design embodiments of the invention with varied dimensions.

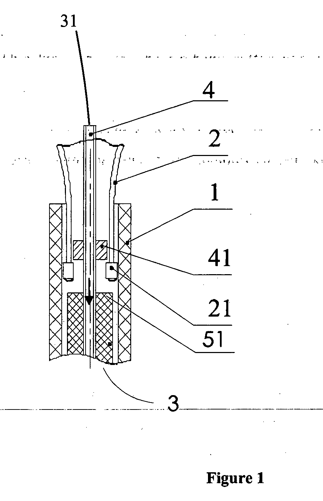

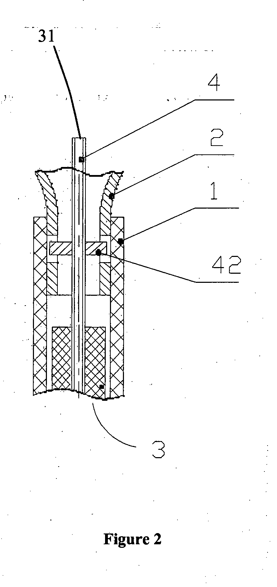

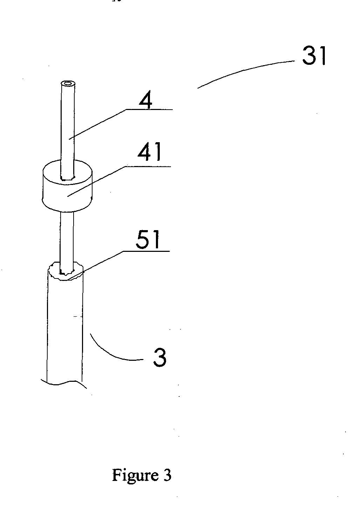

FIG.1 and FIG.2 illustrate two exemplary embodiments of a neurovascular self expanding stent delivery apparatus of the current invention. In FIG.1 there is seen a distal section **4** and a middle section **3** of an inner shaft **31.** Attached to the inner shaft **31** there is a first blocking member **41.** FIG. 3 is partial exploded views of the distal end of the inner shaft 31, wherein the blocking member **41** is a disc-shape disposed on the inner shaft. Returning to FIG. 1, the larger outer diameter of the middle section **3** of inner shaft **31** compared to the outer diameter of the distal section **4** provides a second blocking member **51.** First blocking member **41** and second blocking member **51** are configured along the inner shaft **31** so that the first and second blocking members act in conjunction with one another to form a gap that accepts locking member **21** of a stent **2.** In use, interlocking the stent **2** with the inner shaft **31** by placing the locking member **21** into the gap formed by the first and second blocking members **41, 51** allows for retractable release of said stent **2** from the outer catheter **1** to a neurovascular target site.

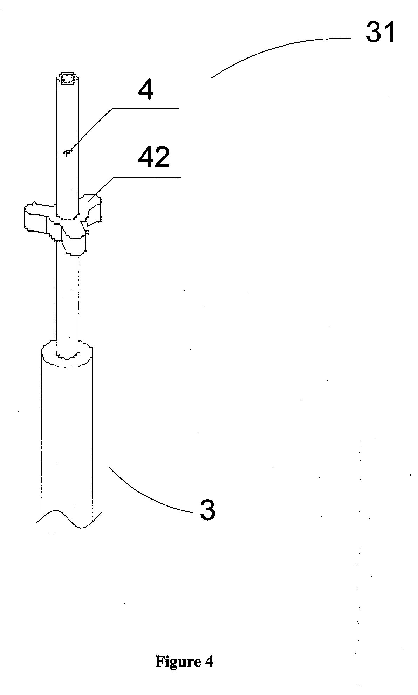

FIG.2 illustrates another preferred embodiment of self expanding stent delivery apparatus of the current invention. In FIG.2, there is seen a distal section and a middle section of an inner shaft **31.** Attached to the inner shaft **31** there is a first blocking member **42.** In this embodiment, the first blocking member **42** is shown protruding through the stent **2.** In this way, the blocking member **42** interlocks with the stent **2** and allows for retractable release of said stent **2** from the outer catheter **1.** Exemplary first blocking members **42** that can be used with this embodiment include a gear-shaped member, some of which are shown in figures 4-6. When the first blocking member **42** is a gear-shaped member, the gear-shape can be configured to have at least 2 teeth, more preferably from 2 teeth to 8 teeth. The number, the size, the shape and the placement of the teeth for the gear-shaped first blocking member **42** are preferably configured to fit within the open space of the cells of a stent. FIGS. 4, 5 and 6 are partial exploded views of the distal end of the inner shaft. The blocking members **42** disposed on the inner shaft takes the gear form in three, four and six teeth configurations respectively.

For use with an inner shaft of the current invention, wherein said inner shaft is configured with at least one blocking member to interlock the inner shaft with the stent, there is described a stent. Preferably, the stent is a neurovascular stent; more preferably a collapsible, self-expanding neurovascular stent; more preferably still a collapsible, self-expanding, closed-cell neurovascular stent. Such stents and methods for constructing such stents are known in the art. Preferably, the stent is configured to be pre-loaded on the inner shaft of the current invention and within the distal portion of the outer catheter so as to be retractable when used with a apparatus comprising an inner shaft of the current invention. The purpose of radio-opaque markers is to allow a user to identify the location of a marked object when said object is *in vivo*. Thus, a radiopaque marker on outer catheter **1** will allow for visualization of the catheter relative a target delivery location. Further, a radiopaque marker on the stent **2** will allow for visualization of the stent relative a target location. When the inner shaft **31,** the stent **2** and the outer catheter **1** are all marked with a radiopaque marker, the relative positioning of these devices one to the other is more easily determined.

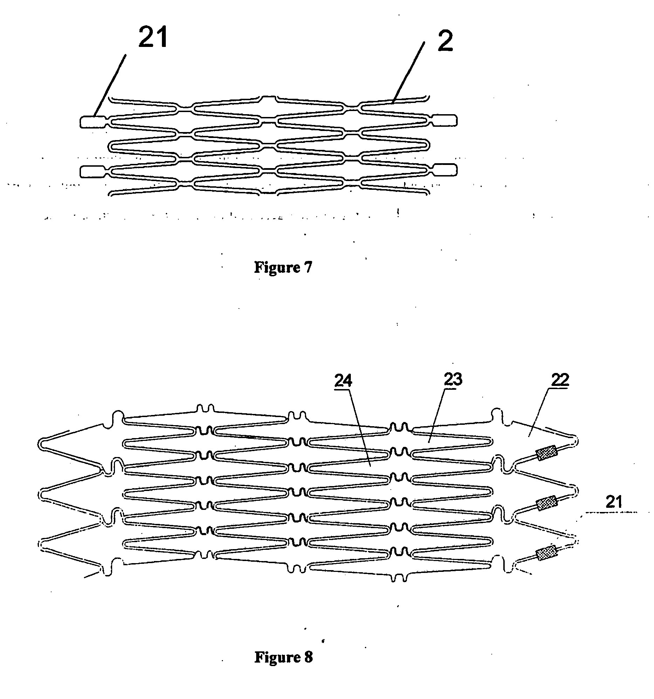

FIG. 7 illustrates a self expanding stent **2** comprising locking members **21** at the most proximal end. Locking members **21** are preferably placed on the proximal end of a self-expanding stent **2.** More preferably, stent **2** will comprise at least two locking members **21.** Most preferably, stent **2** will comprise a plurality of proximal legs, each of said proximal legs comprising a locking member **21.** Preferably, said self-expanding stent is preloaded on the inner shaft **31** and within the distal end of an outer catheter **1.** The length of inner shaft is preferably about 2-50cm longer than outer catheter 1. Turning back to FIG.1, there is a self-expanding stent **2** comprising a locking member **21** as illustrated in Fig.7, and the stent is showed interlocked with the inner shaft 31. In FIG.1 stent **2** is shown in releasable contact with inner shaft **31** and partially delivered from within the distal section of outer catheter **1** wherein locking member **21** is positioned between blocking members **41** and **51.** In such an arrangement, the stent **2** can be partially delivered out of catheter **1** by pulling the outer catheter **1** while the inner shaft **31** remains stationary. This movement applies a force between blocking members **51** and locking member **21.** So long as the stent **2** has not been fully released out of catheter **1,** then stent **2** can be retracted back into catheter **1** by keeping the outer catheter 1 stationary while the inner shaft **31** is pulled, thereby applying force between blocking members **41** and locking member **21** to retract the stent **2.** Confinement of locking member **21** within the inner diameter of catheter **1** provides a force to cause stent **2** to remain in a collapsed position, particularly so at its proximal end wherein the locking member **21** resides. This confinement provides for locking member **21** to remain positioned in the gap between blocking members **41** and **51.** Once stent **2** is fully delivered from within the inner diameter of catheter **1,** the stent will expand, thus removing locking member **21** from its position within the gap of blocking members **41** and **51.**

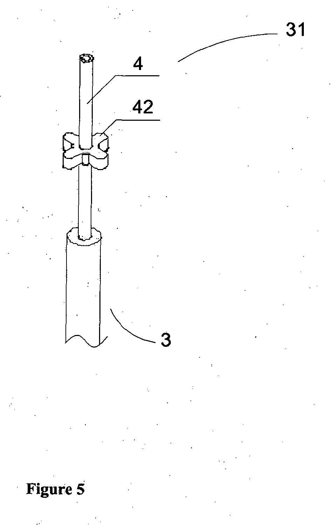

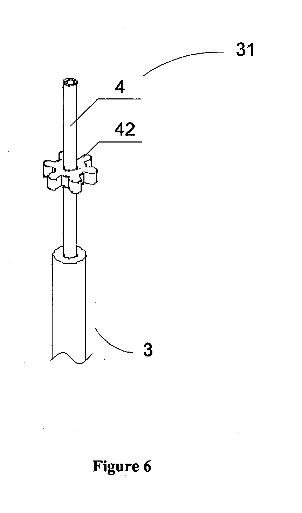

FIG. 8 illustrates a self expanding stent comprising locking members near the proximal end. At least one stent marker **21** is attached to the proximal stent strut. The radiopaque marker **21** thickness will be slightly larger than that of stent strut. The stent cell **22** at the proximal end is larger than the stent cells **23, 24** in the middle, so as for the blocking member **42** fixed in the inner shaft **31** to be easily interlocked together with. As illustrated in Fig. 4, first blocking member **42** has 3 gear teeth; as illustrated in Fig 5, first blocking member **42** has 4 gear teeth; as illustrated in Fig. 6, first blocking member **42** has 6 gear teeth. First blocking member **42** will preferably have 2-8 gear teeth. For instance, a gear with 2, 3 or 6 teeth could be used for six crown design stents, and a gear with 2 or 4 teeth could be used for eight crown design stents. The outside diameter of the first blocking member **42** shall be smaller than then inner diameter of the outer catheter **1.** First blocking member **42** teeth could be fabricated in different geometry shapes. Turning back to FIG.2, there is a self-expanding stent **2** as illustrated in Fig.8. The first blocking member **42** gear teeth are interlocked with stent **2** in the area of stent cell **22.** In such an arrangement, the stent **2** can be released out of catheter **1** by pulling the outer catheter **1** while the inner shaft **31** remains stationary. So long as the stent **2** has not been fully released out of catheter **1,** then stent **2** can be retracted back into catheter **1.** When the physician wants to "pull" the partial expanded stent back into the outer catheter **1,** inner shaft **31** is pulled proximately, and the gear teeth of the first blocking member **42** will engage stent **2** in the area of stent cell **22** by coming into contact with the strut members of the cell, and force the stent **2** to retreat back into the outer catheter **1.** As a result, stent **2** will be recaptured within the outer catheter **1.** To treat wide neck aneurysm, preferably, stent is covered with graft material in the middle, but not on the ends (not shown in Fig.8). Graft in the middle of the stent will efficiently cover aneurysm wide neck, while the stent end without being graft covered will be interlocked with the blocking members on the inner shaft. Stent graft material is made of materials such as e-PTFE, Polyurethane, etc. The first blocking member **42** fixed on the distal section 4 of the inner shaft **31** will interlock with cells at a more proximal end of stent **2** wherein the stent is not covered by graft material, such that graft material will not be damaged by first blocking member **42.**

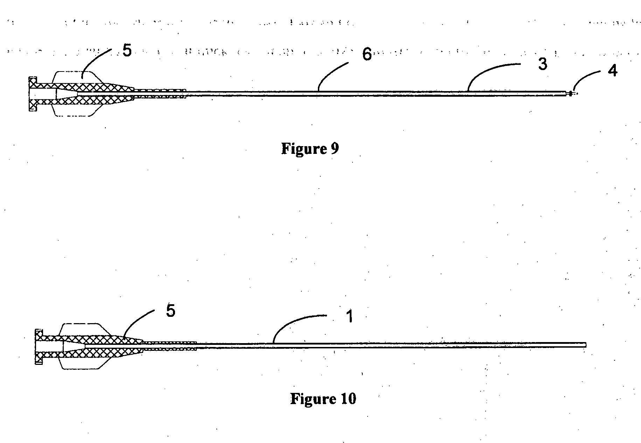

As is better seen in FIG.9, the inner shaft is designed for navigating tortuous paths, such as a tortuous neurovascular path, to deliver a self-expanding stent. The inner shaft **31** is designed to have a plurality of sections wherein said sections provide both strength and flexibility so as to deliver a stent through a tortuous neurovascular path and without damaging the stent. In order to achieve this objective, inner shaft **31** is generally constructed as follows. This proximal section 6 is preferably a metal hypotube material made of materials such as nintinol, stainless steel. The inner diameter of inner shaft **31** should be a sufficient dimension to allow smooth passage of a guidewire. The inner diameter may further comprise a lubricious inner liner to aid in smooth passage of the guidewire. Inner shaft **31** is further constructed to comprise a middle section **3** that provides exceptional flexibility, trackability and kink resistance. This middle section is preferably made of materials such as polyether block amide material, and, optionally, is a polyether block amide material over a braided metal wire reinforcement. Inner shaft **31** is further constructed to comprise a distal section **4** that is of a length within the range of sizes from about 3 cm to about 5 cm. This distal section is flexible and strong material, preferably a polyimide material, and has an outer diameter of about 0.42 mm (0.0165 inch) and an inner diameter that is sufficiently sized to allow smooth passage of a 0.36 mm (0.014 inch) guidewire. Inner shaft **31** also preferably comprises a radio-opaque marker, which is preferably, but not necessarily, located on first blocking member **41** or **42.**

As is better seen in FIG.10, the outer shaft **1** is designed for navigating tortuous paths, such as a tortuous neurovascular path, to deliver a self-expanding stent. The outer shaft **1** is bonded to hub **5.** In one preferred embodiment, the outer catheter 1 is configured to provide a plurality of sections. The proximal section is preferably a rigid stiffness polyether block amide, such as Pebax® 7233, and covers a braided wire reinforcement. The inner diameter should be a sufficient dimension to allow for smooth passage of an inner shaft **31** as described herein. More preferably, the inner diameter should be of a sufficient dimension to allow for smooth passage of an inner shaft **31** as described herein and further comprising a preloaded compressed closed cell neurovascular stent for delivery to a target location. The inner diameter may further comprise a lubricious inner liner to aid in smooth passage of the inner shaft and/or preloaded stent. In one example, such an inner liner is constructed from a PTFE material having at least a 0.025 mm (0.001 inch) wall thickness. Outer catheter **1** further comprises a middle section. The middle section is preferably a medium stiffness polyether block amide, such as Pebax® 5533, and covers a braided wire reinforcement. The inner diameter of this middle section may further comprise a PTFE liner having a wall thickness of about 0.025 mm (0.001 inch).

Turning to Fig. 10, there is shown an outer catheter **1** designed for navigating tortuous pathways to deliver a self-expanding stent. In the figure outer catheter **1** is bonded to hub **5,** Outer catheter **1** provides a proximal section, middle section and a distal section. In an exemplary embodiment, the proximal section is about 93 cm in length and comprises a rigid stiffness polyether amide such as Pebax 7233 covering a braided wire reinforcement. The braided wire reinforcement is preferably about 0.025 mm (0.001 inch) in height, about 0.076 mm (0.003 inch) in length and has a braid density of about 24-39 ± 2 picks/cm (60-100 ±5 ppi). The proximal section has an outer diameter of about 0.99 mm (0.039 inch) and an inner diameter of about 0.74 mm (0.029 inch), thereby allowing for smooth passage through a tortuous path and retractable deployment of an inner shaft comprising a preloaded self expanding closed cell neurovascular stent. The inner portion of the outer catheter **1** preferably comprises a lubricious inner liner to aid in smooth passage of the inner shaft and preloaded stent. One exemplary lubricious liner is PTFE material having a wall thickness of about 0.025 mm (0.001 inch). In an exemplary embodiment, the middle section is about 23 cm in length, has an outer diameter of about 0.94 mm (0.037 inch), has an inner diameter of about 0.74 mm (0.029 inch), and comprises a medium stiffness polyether block amide such as Pebax 5533 covering a braided wire. Preferably the braided wire is 0.025 mm (0.001 inch) by 0.076 mm (0.003 inch) with a 24-39 ± 2 picks/cm (60-100 ±5 ppi) braid density. Preferably, the inner portion of the outer catheter middle section comprises a lubricious liner such as PTFE with a wall thickness of about 0.025 mm (0.001 inch). In an exemplary embodiment, outer catheter **1** comprises a distal section that is about 20 cm in length, has an outer diameter of about 0.94 mm (0.037 inch), and has an inner diameter of about 0.74 mm (0.029 inch). The distal section is preferably a soft stiffness polyether block amide, such as Pebax® 2533 covering a braided wire reinforcement, as described. The inner portion of this distal section may further comprise a PTFE liner having a wall thickness of about 0.025 mm (0.001 inch). Outer catheter **1** also preferably comprises at least one radio-opaque marker, preferably located at the distal end of said outer catheter **1.**

In one embodiment, the invention provides a neurovascular stent delivery apparatus, wherein said delivers apparatus includes an outer catheter, and an inner shaft located coaxially within the outer catheter; a stent is mounted on the distal section of the inner shaft and preloaded within the outer catheter distal region, wherein the inner shaft includes at least one stent blocking member disposed in the distal section, wherein the self-expanding stent has proximal and distal end and is comprised of a plurality of closed cells and further includes locking members, wherein the blocking members fixed on the inner shaft form a gap that accepts the locking member of the self-expanding stent and allows for retractable release of said stent back into the outer catheter, wherein optionally, the blocking member on the inner shaft is in gear form with various number of teeth, such that the blocking member is embedded within the said stent cell in a configuration that allows the blocking member fixed on the inner shaft to engage the stent when the inner shaft is pulled proximately, and wherein one or both of the stent and the first blocking member comprises a radio-opaque marker.

Preferred features are as follows:

The self-expanding stent may be covered with graft material at a portion of the stent selected from: the proximal portion and distal portion; or the proximal portion, middle portion and distal portion. The graft material may be made of e-PTFE or Polyurethane.

The blocking member may be gear-shaped and have a plurality of teeth ranging from 2 to 8 and be configured to accommodate a stent cell design. The blocking member may be made of radioopaque materials such as metal, metal alloy, or radiopaque polymers.

The stent cells may be larger at the ends of the stent than in the middle.

The outer catheter may be designed with a material of differing stiffness from proximal end to distal end, distal end being the softest, and wherein the differing stiffness of the outer catheter may be configured for navigating tortuous paths, such as a tortuous neurovascular path, to deliver a self-expanding stent

One or more of the radiopaque markers may be made of gold, silver, platinum or its alloy.

The stent may have at least one radiopaque marker at the proximal ends.

The blocking members may be radiopaque.

The outer catheter may cover a braided wire or coil reinforcement.

In another embodiment, the invention provides a neurovascular stent delivery apparatus, wherein said delivery apparatus includes an outer catheter and an inner shaft located coaxially within the outer catheter; a compressed self-expanding stent comprising a plurality of closed cells mounted on the distal section of the inner shaft and preloaded within the outer catheter distal region; wherein the inner shaft comprises one or more blocking members disposed in the distal section.

Preferred features are as follows:

At least one of the one or more blocking members may be made of materials such as metal, metal alloy, or radiopaque polymers.

The self-expanding stent may comprise at least one locking member. The locking members may be made of radiopaque materials.

The inner shaft may comprise two or more blocking members, the first blocking member having a disc-shape and being located on the inner shaft at a more distal position relative to the second blocking member such that the first and second blocking members are configured to provide a gap between the first and second blocking members, the gap being sufficient to receive a locking member portion of the compressed self-expanding stent.

A first of the one or more blocking members may have a gear-shape comprising a plurality of teeth configured to interlock with the cells of said compressed self-expanding stent.

The self-expanding stent may be covered with a graft material.

The inner shaft may be made of material of varying hardness, wherein said distal section is softest.

The outer catheter may be made of material of varying hardness, wherein said distal section is softest.

The cells of the self-expanding stent may be larger at the proximal ends compared to the cells of the middle section of the stent.

A suitable method for using the apparatus of the invention and which is for retractably delivering a self-expanding stent to a neurovascular target site comprises the steps of:
a. obtaining a neurovascular delivery apparatus containing a preloaded and compressed self expanding stent, wherein said preloaded and compressed self expanding stent is interlocked with an inner shaft member of said neurovascular delivery apparatus;
b. navigating a neurovascular path towards a neurovascular target site;
c. partially delivering said preloaded and compressed self-expanding stent by pulling the outer catheter to partially expose the stent out of the distal end of an outer catheter member of a neurovascular delivery device;
d. optionally, retracting said partially released stent back inside of said outer catheter distal end; and
e. fully delivering said preloaded and compressed self-expanding stent to said target site by pulling the outer catheter to expose the stent out of the distal end of an outer catheter member of a neurovascular delivery device.

Preferably features of the method are as follows:

The neurovascular delivery apparatus may comprise an inner shaft having a first blocking member and a second blocking member configured along said inner shaft to form a gap section that accepts the locking member of said preloaded and compressed self expanding stent.

The stent may be partially deployed from said outer catheter distal end by pulling the outer catheter proximately, while the inner shaft is held in a fixed position.

The partially deployed stent may be pulled back inside of said outer catheter by applying a force to the inner shaft that cause the first blocking member to contact the locking member of said stent and retract the said stent back into said outer catheter.

The neurovascular delivery apparatus may comprise an inner shaft having a first blocking member configured as a gear with a plurality of teeth that interlocks with the cells of said preloaded and compressed self expanding stent. The plurality of teeth may be at least 2, or may be selected from the group consisting of 2, 3, 4, 5, 6, 7 and 8.

The stent may be partially deployed from said outer catheter distal end by pulling the outer catheter proximately, while the inner shaft is held in a fixed position.

The partially deployed stent may be pulled back inside of said outer catheter by applying a force to the inner shaft that causes said plurality of teeth to contact the strut portion of the cell and retract the said stent back into said outer catheter.

The cells may be larger at the proximal end of said self-expanding stent compared to the middle section cells.

## Claims

1. A neurovascular stent delivery apparatus, wherein said delivery apparatus includes an outer catheter (1) and an inner shaft (31) located coaxially within the outer catheter (1); a compressed self-expanding stent (2) comprising a plurality of closed cells mounted on the distal section (4) of the inner shaft (31) and preloaded within the outer catheter distal section (4); wherein the inner shaft (31) comprises two or more blocking members (41, 51) disposed in the distal section (4), the first blocking member (41) having a disc-shape and being located on the inner shaft (31) at a more distal position relative to the second blocking member (51) such that the first and second blocking members are configured to provide a gap between the first and second blocking members, the gap being sufficient to receive a locking member portion (21) of the compressed self-expanding stent (2), wherein the first blocking member (41) having a disc-shape is located only at the proximal end of the stent (2), and wherein the inner shaft (31) has an inner diameter which allows passage of a guidewire.

2. A neurovascular stent delivery apparatus, wherein said delivery apparatus includes an outer catheter (1) and an inner shaft (31) located coaxially within the outer catheter (1); a compressed self-expanding stent (2) comprising a plurality of closed cells mounted on the distal section (4) of the inner shaft (31) and preloaded within the outer catheter distal region (4); wherein the inner shaft (31) comprises one or more blocking members (42) disposed in the distal section (4), wherein a first of the one or more blocking members (42) has a gear-shape comprising a plurality of teeth configured to interlock with the cells of said compressed self-expanding stent (2), and wherein, in use, the stent (2) is fully deployed from the outer catheter distal end (4) by pulling the outer catheter (1) proximately which causes the plurality of teeth to contact a strut portion of the cells until the stent (2) is completely outside of the outer catheter (1), wherein the stent (2) expands thereby removing the cells from the proximity of the gear teeth, and wherein the cells of the self-expanding stent (2) are larger at the proximal end of the stent compared to the cells of the middle section of the stent in order for the stent (2) to be interlocked with the gear-shaped blocking member (42) on the inner shaft (31).

3. The delivery apparatus of claim 1, wherein the locking member portion (21) is made of a radiopaque material.

4. The delivery apparatus of any preceding claim, wherein the self-expanding stent (2) is covered with a graft material.

5. The delivery apparatus of any preceding claim, wherein at least one of the blocking members is made of a radiopaque material.

6. The delivery apparatus of any preceding claim, wherein said inner shaft (31) is made of material of varying hardness, wherein said distal section is the softest.

7. The delivery apparatus of any preceding claim, wherein said outer catheter (1) is made of material of varying hardness, wherein said distal section is the softest.

8. The neurovascular stent delivery apparatus of any preceding claim, wherein the outer catheter (1) covers a braided wire or coil reinforcement.

9. The neurovascular stent delivery apparatus of claim 1, wherein the stent (2) has at least one radiopaque marker at the proximal ends.

10. The neurovascular stent delivery apparatus of any preceding claim for use in therapy.

11. The neurovascular stent delivery apparatus of any one of claims 1 to 9 for use for retractably delivering a self-expanding stent to a neurovascular target site.

## Patentansprüche

1. Abgabevorrichtung für neurovaskulären Stent, wobei die Abgabevorrichtung umfasst: einen äußeren Katheter (1) und einen in dem äußeren Katheter (1) koaxial angeordneten inneren Schaft (31), einen komprimierten selbstaufweitenden Stent (2) mit einer Vielzahl von geschlossenen Zellen, der an dem distalen Abschnitt (4) des inneren Schafts (31) angebracht ist und in dem distalen Abschnitt (4) des äußeren Katheters vorgespannt ist, wobei der innere Schaft (31) zwei oder mehr Blockierelemente (41, 51) umfasst, die in dem distalen Abschnitt (4) angeordnet sind, wobei das erste Blockierelement (41) scheibenförmig ausgebildet ist und an einer distaleren Position als das zweite Blockierelement (51) auf dem inneren Schaft (31) angeordnet ist, so dass das erste und das zweite Blockierelement derart konfiguriert sind, dass zwischen dem ersten und dem zweiten Blockierelement eine Lücke gebildet ist, wobei die Lücke ausreicht, um einen Arretierelementabschnitt (21) des komprimierten selbstaufweitenden Stents (2) aufzunehmen, wobei das erste Blockierelement (41) mit der scheibenförmigen Ausbildung nur am proximalen Ende des Stents (2) angeordnet ist und wobei der innere Schaft (31) einen Innendurchmesser aufweist, der die Durchführung eines Führungsdrahtes ermöglicht.

2. Abgabevorrichtung für neurovaskulären Stent, wobei die Abgabevorrichtung einen äußeren Katheter (1) und einen in dem äußeren Katheter (1) koaxial angeordneten inneren Schaft (31) umfasst, einen komprimierten selbstaufweitenden Stent (2) mit einer Vielzahl von geschlossenen Zellen, der an dem distalen Abschnitt (4) des inneren Schafts (31) angebracht ist und in dem distalen Bereich (4) des äußeren Katheters vorgespannt ist, wobei der innere Schaft (31) ein oder mehr Blockierelemente (42) umfasst, die in dem distalen Abschnitt (4) angeordnet sind, wobei ein erstes der ein oder mehr Blockierelemente (42) zahnradförmig ausgebildet ist und eine Mehrzahl von Zähnen aufweist, die dafür konfiguriert sind, mit den Zellen des komprimierten selbstaufweitenden Stents (2) zu verzahnen, und wobei der Stent (2) beim Gebrauch vollständig aus dem distalen Ende (4) des äußeren Katheters ausgefahren wird, indem der äußere Katheter (1) in proximaler Richtung herangezogen wird, was bewirkt, dass die Mehrzahl von Zähnen mit einem Verstrebungsabschnitt der Zellen in Kontakt kommt, bis sich der Stent (2) vollständig außerhalb des äußeren Katheters (1) befindet, wobei sich der Stent (2) dadurch aufweitet, wodurch sich die Zellen aus der Nähe der Zahnradzähne entfernen, und wobei die Zellen des selbstaufweitenden Stents (2) an dem proximalen Ende des Stents größer sind als die Zellen des mittleren Abschnitts des Stents, damit sich der Stent (2) mit dem zahnradförmigen Blockierelement (42) an dem inneren Schaft (31) verzahnen lässt.

3. Abgabevorrichtung nach Anspruch 1, wobei der Arretierelementabschnitt (21) aus einem strahlenundurchlässigen Material besteht.

4. Abgabevorrichtung nach einem der vorhergehenden Ansprüche, wobei der selbstaufweitende Stent (2) mit einem Transplantatmaterial bedeckt ist.

5. Abgabevorrichtung nach einem der vorhergehenden Ansprüche, wobei mindestens eines der Blockierelemente aus einem strahlenundurchlässigen Material besteht.

6. Abgabevorrichtung nach einem der vorhergehenden Ansprüche, wobei der innere Schaft (31) aus Material mit unterschiedlicher Härte besteht, wobei der distale Abschnitt am weichsten ist.

7. Abgabevorrichtung nach einem der vorhergehenden Ansprüche, wobei der äußere Katheter (1) aus Material mit unterschiedlicher Härte besteht, wobei der distale Abschnitt am weichsten ist.

8. Abgabevorrichtung für neurovaskulären Stent nach einem der vorhergehenden Ansprüche, wobei der äußere Katheter (1) eine Drahtgefecht- oder Spiralverstärkung überdeckt.

9. Abgabevorrichtung für neurovaskulären Stent nach Anspruch 1, wobei der Stent (2) mindestens einen strahlenundurchlässigen Marker an den proximalen Enden aufweist.

10. Abgabevorrichtung für neurovaskulären Stent nach einem der vorhergehenden Ansprüche zur Verwendung in der Therapie.

11. Abgabevorrichtung für neurovaskulären Stent nach einem der Ansprüche 1 bis 9 zur Verwendung zur zurückziehbaren Abgabe eines selbstaufweitenden Stents an einen neurovaskulären Zielort.

## Revendications

1. Appareil permettant la mise en place d'un stent neurovasculaire, cet appareil comprenant un cathéter externe (1) et une tige interne (31) positionnée coaxialement dans le cathéter externe (1), un stent autoextensible comprimé (2) comprenant un ensemble de cellules fermées monté sur le segment distal (4) de la tige interne (31) et précontraint dans le segment distal (4) du cathéter externe, la tige interne (31) comprenant deux ou un plus grand nombre d'éléments de blocage (41, 51) montés dans le segment distal (4), le premier élément de blocage (41) ayant la forme d'un disque et étant situé sur la tige interne (31) dans une position plus distale par rapport au second élément de blocage (51) de sorte que le premier et le second éléments de blocage soient conformés pour définir un intervalle entre eux, cet intervalle étant suffisant pour recevoir une partie d'élément de verrouillage (21) du stent auto-extensible comprimé (2), le premier élément de blocage (41) ayant la forme d'un disque étant positionné uniquement à l'extrémité proximale du stent (2) et la tige interne (31) ayant un diamètre interne qui permet le passage d'un fil de guidage.

2. Appareil permettant la mise en place d'un stent neurovasculaire, cet appareil comprenant un cathéter externe (1) et une tige interne (31) positionnée co-axialement dans le cathéter externe (1), un stent auto-extensible comprimé (2) comprenant un ensemble de cellules fermées monté sur le segment distal (4) de la tige interne (31) et précontraint dans la section distale (4) du cathéter externe, la tige interne (31) comprenant un ou plusieurs éléments de blocage (42) situés dans le segment distal (4), un premier élément de blocage (42) ayant la forme d'un engrenage comprenant un ensemble de dents conformées pour s'enclencher dans les cellules du stent auto-extensible comprimé (2), et, lors de l'utilisation, le stent (2) étant totalement déployé à l'extérieur de l'extrémité distale (4) du cathéter externe en tirant le cathéter externe (1) vers l'extrémité proximale, de manière à mettre en contact l'ensemble de dents avec une partie faisant saillie des cellules jusqu'à ce que le stent (2) soit situé complètement à l'extérieur du cathéter externe (1), le stent (2) s'étendant alors en éloignant les cellules de la proximité des dents d'engrenage, et les cellules du stent auto-extensible (2) étant plus grandes au niveau de l'extrémité proximale du stent par comparaison avec les cellules du segment médian du stent de façon à permettre l'enclenchement du stent (2) avec l'élément de blocage en forme d'engrenage (42) sur la tige interne (31).

3. Appareil conforme à la revendication 1, dans lequel la partie d'élément de blocage (21) est réalisée en un matériau radio-opaque.

4. Appareil conforme à l'une quelconque des revendications précédentes, dans lequel le stent auto-extensible (2) est recouvert d'un matériau greffé.

5. Appareil conforme à l'une quelconque des revendications précédentes, dans lequel au moins l'un des éléments de blocage est réalisé en un matériau radio-opaque.

6. Appareil conforme à l'une quelconque des revendications précédentes, dans lequel la tige interne (31) est réalisée en un matériau de dureté variable, le segment distal étant le plus mou.

7. Appareil conforme à l'une quelconque des revendications précédentes, dans lequel le cathéter externe (1) est réalisé en un matériau de dureté variable, le segment distal étant le plus mou.

8. Appareil permettant la mise en place d'un stent neuro-vasculaire conforme à l'une quelconque des revendications précédentes dans lequel le cathéter externe (1) recouvre un serpentin ou un fil tressé de renfort.

9. Appareil permettant la mise en place d'un stent neuro-vasculaire conforme à la revendication 1, dans lequel le stent (2) comprend au moins un repère radio opaque à ses extrémités proximales.

10. Appareil permettant la mise en place d'un stent neuro-vasculaire conforme à l'une quelconque des revendications précédente destiné à être utilisé en thérapie.

11. Appareil permettant la mise en place d'un stent neuro-vasculaire conforme à l'une des revendications 1 à 9, destiné à être utilisé pour permettre la mise en place rétractable d'un stent auto-extensible sur un site neuro-vasculaire cible.
